(19) Europäisches Patentamt
European Patent Office
Office européen des brevets

(11) EP 3 804 610 A1

(12) EUROPEAN PATENT APPLICATION
published in accordance with Art. 153(4) EPC

(43) Date of publication:
14.04.2021 Bulletin 2021/15

(51) Int Cl.:
A61B 5/00 (2006.01)    G01N 33/68 (2006.01)
G01N 33/60 (2006.01)

(21) Application number: 19812464.6

(22) Date of filing: 29.05.2019

(86) International application number:
PCT/KR2019/006449

(87) International publication number:
WO 2019/231238 (05.12.2019 Gazette 2019/49)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 29.05.2018 KR 20180061200

(71) Applicant: Seoul National University R & DB
Foundation
Seoul 08826 (KR)

(72) Inventors:
• KIM, Sang Jeong
Seoul 04426 (KR)
• CHUNG, Gee Hoon
Seoul 03082 (KR)
• KIM, Chae Young
Seoul 06297 (KR)

(74) Representative: Berggren Oy, Tampere
Visiokatu 1
33720 Tampere (FI)

(54) METHOD FOR MEASURING INTENSITY OF PAIN

(57) The present disclosure relates to a method for measuring the intensity of pain and, more particularly, provides a method capable of measuring the intensity of pain by providing a pain template.

【Fig. 4e】

EP 3 804 610 A1

## Description

### [TECHNICAL FIELD]

The present disclosure relates to a method for objectively measuring the intensity of pain and, more particularly, provides a method capable of objectively measuring the intensity of pain by providing a pain template.

### [BACKGROUND ART]

Pathological pain has no survival benefit unlike natural physiological pain, and is mainly caused by abnormalities in the nervous system. Neuropathic pain is a typical pathological pain, and individuals with neuropathic pain generally perceive harmless external sensory stimuli as harmful and feel pain. Research on neuropathic pain has been actively conducted over the last few decades, but currently, no standard method has been established for objectively assessing the intensity of pain. Since chronic pain symptoms and pain intensity after nerve damage depend on the individual, it is difficult to develop a standard assessment method for objectively assessing this. The degree to which pain is amplified depends on the individuals, and objective diagnosis methods have not been established, so at present, there is no choice but to rely on the patient's own subjective statement in measuring the patient's pain level.

### [DETAILED DESCRIPTION OF THE INVENTION]

### [Technical Problem]

Traditional techniques aimed at diagnosing neuropathic pain have relied primarily on patient self-report and several physical diagnosis methods. Traditional tools used to screen a neuropathic pain include: Michigan Neuropathy Screening Instrument, Neuropathic Pain Scale, Leeds Assessment of Neuropathic Symptoms and Signs, Neuropathic Pain Questionnaire, Neuropathic Pain symptom Inventory, "Douleur Neuropathique en 4 questions", pain DETECT, Pain Quality Assessment Scale, Short-Form McGill Pain Questionnaire. While there are some differences, all the above-mentioned techniques rely on self-reporting of pain felt by individuals in response to sensory stimuli.

A standardized method for quantitative measurement of neuropathic pain mostly used in clinical practice is the method introduced in 2006 by German Research Network on Neuropathic Pain (Deutscher Forschungsverbund Neuropathischer Schmerz [DFNS]). A sensory stimulus is applied to the skin to record the intensity of pain felt by an individual, wherein different types of external stimulus is applied to the individual by changing the intensity, thereby recording whether the individual suffers from pain. Among them, von Frey filaments, graded pinprick stimulation, and pressure stimulation are used as mechanical stimulation. Von Frey filaments use filaments of different thickness to apply stimulation, wherein different bending forces are generated according to the thickness of the filament, and the intensity of stimulation is divided into grades. This is a suitable method for precisely controlling the amount of stimulus applied.

Traditional methods are difficult to distinguish false reports from patients due to the patient self-reporting nature. Also, it is not possible to record the severity of pain due to stimulation in patients or children with difficulty in linguistic communication, or individuals with weak cognitive function. Even if using methods of recording only behavioral changes, such as paw withdrwal to certain levels of external stimuli, without linguistic communication, it cannot be applied to unconscious patients or patients with impaired motor function.

### [Technical Solution]

Therefore, the present inventors have intended to provide a pain template using the expression pattern of an indicator substance, and a method capable of objectively measuring the intensity of pain by using the pain template.

One aspect of the present disclosure provides a method for objectively measuring the pain level through images of the brain without depending on linguistic communication or behavioral response.

Another aspect of the present disclosure relates to generating a pain template using an expression pattern of an indicator substance according to the intensity of pain, and method of measuring the pain intensity of a target individual using the pain template. Specifically, the present disclosure provides a method of measuring the intensity of pain in a test individual by analyzing a correlation between the pain indicator substance and the pain intensity in the brain of the reference individual to generate a pain template, and applying the expression pattern of the indicator substance measured in the test individual to the pain template.

More specifically, the present disclosure relates to a method for measuring pain intensity comprising the steps of:

generating a pain template that indicates a corerelation between an availability of an indicator substance measured at each pain intensity in at least two or more brain regions of a reference individual, and each brain region and each stage of pain intensity; and

applying the availability of the indicator substance measured in the at least two brain regions of the test individual to the pain template, selecting the stage of the pain intensity having the highest similarity with the availability of the indicator substance of each stage of the pain intensity through similarity analysis, to determining the pain intensity of the test individual.

The at least two or more brain regions are regions in which the availability of the indicator substance changes due to pain, and may be two more selected from the group consisting of: (1) rostral caudate-putamen of striatum, left, (2) caudal caudate-putamen of striatum, left, (3) insular cortex, left, (4) secondary somatosensory cortex, left, (5) Hippocampus, right; rostral, (6) Hippocampus, right; caudal, (7) primary somatosensory cortex, left; trunk region, (8) primary somatosensory cortex, right; trunk region, (9) primary somatosensory cortex, right; hindlimb region, (10) secondary somatosensory cortex, right, (11) Hypothalamus, right; posterior nucleus, and (12) Anterior midcingulate cortex.

The pain template is generated by performing a step of standardizing with dividing the availability of indicator substances for at least two or more brain region by a mean value of the availability of the indicator substance for each brain region; and a step of subdividing the standardized value into at least 200 stages of pain intensity through regression analysis, and the mean value for each brain region is the mean value of the availability of the indicator substance for each brain region obtained from a painless control group.

The similarity analysis may be performed by one or more analysis methods selected from the group consisting of Pearson's correlation coefficient analysis, Spearman's correlation coefficient analysis, Euclidean distance analysis, Mahalanobis distance analysis, Support vector analysis, Cosine distance analysis, Manhattan distance analysis, Jaccard coefficient analysis, and Extended Jaccard coefficient analysis, but is not limited thereto.

The Pearson's correlation coefficient analysis is performed by the following Mathematical Formula 2, and the similarity may be evaluated to an extent where the r value calculated by Mathematical Formula 2 is close to 1.

[Mathematical Formula 2]

$$\frac{\sum_{i=1}^{n}(x_i - \bar{x})(y_i - \bar{y})}{\sqrt{\sum_{i=1}^{n}(x_i - \bar{x})^2 \sum_{i=1}^{n}(y_i - \bar{y})^2}}$$

X: standardized availability of indicator substances measured in brain regions of test individuals
Y: availability of indicator substances possessed by any one pain stage of the pain template
$\bar{x}$ : sample mean of X
$\bar{y}$: sample mean of Y
n: number of brain regions

Hereinafter, the present disclosure will be described in more detail.

The present disclosure provides a method for measuring the pain intensity of a test individual by analyzing a correlation between an indicator substance of pain and pain intensity in the brain of a reference individual to generate a pain template, and applying the availability pattern or the expression pattern of the indicator substance measured in the test individual to the pain template. Accordingly, in the present disclosure, the availability or expression pattern of a pain indicator substance in the brain of a reference substrate is analyzed in order to generate a pain template, and the availability or expression pattern of an indicator substance in the test individual is measured and applied to the pain template.

As used herein, the term "availability" of an indicator substance means the amount of the indicator substance that is in a state of being able to bind to a binding substance, and can encompass the expression level or functional activity of the indicator substance in vivo. For example, the availability of the indicator substance can be measured by the expression level of the indicator substance. Alternatively, the availability of the indicator substance can be measured by the activity of the indicator substance.

The indicator substance according to the intensity of pain is a substance present in specific brain regions involved in the processing of pain information, and may be a substance that the availability or expression level of the indicator substance shows a specific pattern depending on the intensity of pain felt by the target individual. Examples of the indicator substance may be metabotropic glutamate receptor 5 (mGluR5). One embodiment of the present disclosure is a method capable of objectively measuring the intensity of pain by using the availability or expression pattern of metabotropic glutamate receptors present in the brain. The availability or expression level of metabotropic glutamate receptors present in specific brain regions involved in the processing of pain information shows a specific pattern depending on the intensity of pain felt by an individual. For example, in the present disclosure, the indicator substance may be a substance that shows changes such as increase or decrease in the brain of an individual. In the present disclosure, the indicator substance may be, for example, metabotropic glutamate receptor 5 (mGluR5), but is not limited thereto.

Metabotropic glutamate receptor 5 (mGluR5) is a type of G-protein-related receptor and is highly expressed in the hippocampus and cerebral cortex. It is known to control neuroplasticity by being mainly distributed in the postsynaptic membrane of nerve cells, and mGluR5 is directly associated with neurological diseases such as fragile X syndrome and neuropathic pain. Diseases related to mGluR5 include pain and drug dependence, neurodegenerative diseases such as amyotrophic lateral sclerosis and multiple sclerosis, Alzheimer's disease, dementia, Parkinson's disease, Hunting pain chorea, psychiatric disorders such as schizophrenia and anxiety, depression, and the like.

Methods for measuring the availability of indicator substances according to the intensity of pain include positron emission tomography (PET), single photon emission computed tomography (SPECT) or the like. Positron emission tomography (PET) in the present disclosure is a method to measure how much the substance exists in a certain area of the body by attaching and injecting a radioactive isotope tracer into a substance that selectively binds to a specific substance present in the body, and then reconstructing the measured radioactive signal as an image through the detector.

In one embodiment of the present disclosure, when mGluR5 is used as an indicator substance according to the intensity of pain, a method of measuring its availability or expression pattern can use PET, SPECT, or the like. For example, the availability or expression pattern of mGluR5 can be measured by binding a labeling substance to ABP688 and tracking the bound labeling substance. In the present disclosure, means for measuring the amount of the indicator substance by specifically binding the tracer substance to the indicator substance may be, for example, a radioactive isotope tracer. For example, the radioactive isotope tracer may be [11C]ABP688, which is a chemical substance that specifically binds to mGluR5, and is capable of measuring mGluR5 availability by tagging a radioactive isotope [11C], but is not limited thereto.

ABP688 is a chemical substance that selectively binds to metabotropic glutamate receptor 5 (mGluR5), and [11C]ABP688 is a substance tagged with radioactive isotope [11C] to ABP688. mGluR5 levels can be measured by performing a Positron Emission Tomography (PET) scan using [11C]ABP688 as a radioactive isotope tracer. Specifically, a radioactive isotope tracer that specifically binds to metabotropic glutamate receptor 5 is injected into a subject, and then metabotropic glutamate receptor 5 in the brain is measured using PET method, and the expression pattern of the individual subject is analyzed, whereby the presence or absence of pain and the pain level can be objectively and precisely measured.

In the present disclosure, the brain region in which the availability of an indicator substance changes due to pain refers to an intracerebral region in which the availability of an indicator substance increases or decreases as pain is applied to an individual. For example, in the present disclosure, a brain region in which the availability of an indicator substance changes due to pain may be a brain region in which the availability of an indicator substance changes due to neuropathic pain induced in the right hind leg.

For example, in the present disclosure, in the case of causing neuropathic pain in the right hind leg, the brain region in which the availability of an indicator substance changes due to pain may be one or two or more selected from the group consisting of:

(1) rostral caudate-putamen of striatum, left (abbreviation: Cpu rostral left),
(2) caudal caudate-putamen of striatum, left (abbreviation: Cpu_caudal_left),
(3) insular cortex, left (abbreviation: Ins left),
(4) secondary somatosensory cortex, left (abbreviation: S2_left),
(5) Hippocampus, right; rostral (abbreviation: Hippo rostral right),
(6) Hippocampus, right; caudal (abbreviation: Hippo _caudal right),
(7) Primary somatosensory cortex, left; trunk region (abbreviation: S1_trunk_left),
(8) Primary somatosensory cortex, right; trunk region (abbreviation: S1_trunk_right),
(9) Primary somatosensory cortex, right; hindlimb region (abbreviation: S1_hindlimb_right),
(10) Secondary somatosensory cortex, right, (abbreviation: S2_right)
(11) Hypothalamus, right; posterior nucleus (abbreviation: Hypothalamus_right), and
(12) Anterior midcingulate cortex (abbreviation: aMCC), but is not limited thereto. Alternatively, the brain region in which the availability of an indicator substance changes due to pain may be 3 or more, 4 or more, 5 or more, 6 or more, 7 or more, 8 or more, 9 or more, 10 or more, 11 or more, or 12 or more selected from the group consisting of (1) to (12) above. Alternatively, the brain region where the availability of indicator substances changes due to pain may be striatum; caudate-putamen, primary somatosensory cortex, secondary somatosensory cortex, and cingulate cortex, but is not limited thereto.

The method for measuring the intensity of pain in a test individual according to the present disclosure includes the step of generating a pain template by analyzing a correlation between the indicator substance of pain and the intensity of pain in the brain of a reference individual.

In more detail, the present disclosure relates to a method for measuring pain intensity comprising the steps of:

generating a pain template that indicates a correlation between an availability of an indicator substance measured at each pain intensity in at least two or more brain regions of the reference individual, and each brain region and each stage of pain intensity; and
applying the availability of the indicator substance measured in the at least two brain regions of the test individual to the pain template, selecting the stage of the pain intensity having the highest similarity by analyzing the availability and similarity of the indicator substance for each stage of the pain intensity through similarity analysis, to determine the pain intensity of the test individual.

In the present disclosure, in order to objectively measure the level of hyperalgesia due to pathological pain, first, a pattern appearing in a brain image was found using a behavioral response. The behavioral technique used the paw withdrawal threshold for von Frey filaments, which is a measurement technique widely used in patient and animal models. After analyzing the brain image pattern for the paw withdrawal threshold, the corresponding brain image pattern can be established as a standard for comparison. After that, when a new brain image is acquired, the brain image is compared with the brain image pattern established as a standard for comparison, so that the pain level of the individual can be measured even without diagnosis such as the paw withdrawal threshold.

In the present disclosure, the individual may be one or more selected from the group consisting of rodents, mice, rats, hamsters, guinea pigs, reptiles, amphibians, mammals, dogs, feline, rabbit neck, pig, cow, sheep, monkeys, primates, non-human mammals, non-human primates, and humans.

In the present disclosure, the reference individual means an individual in which the intensity of pain is already known, and refers to an individual whose pain intensity has been established by biological or statistical methods and can be used as a reference. For example, it may be an individual whose Paw withdrawal threshold was measured by the method of von Frey filaments, but is not limited thereto. For example, in the present disclosure, the reference individual may have induced neuropathic pain in the right hind leg. Alternatively, it may be an individual in which a surgical operation is performed to unilaterally damage nerves and surgically induce neuropathic pain, and after 15 days, the paw withdrawal threshold is measured by the method of von Frey filaments. Inducing neuropathic pain by surgical operation can reduce the tactile threshold and make it more sensitive to stimuli, but the degree of reduction in the threshold is shown to vary from individual to individual, despite being treated in the same surgical operation and experimental environment. In this way, reference individuals with various pain intensities can be obtained. Alternatively, it may be an individual whose pain intensity has been established by linguistic communication, physical diagnosis, behavioral measurement, etc., but is not limited thereto.

In the present disclosure, the pain template refers to a template which measures the availability of an indicator substance in at least two or more regions related to pain among the brain regions of a reference individual, which is an individual whose pain intensity has objectively been established, and shows the availability of an indicator substance by brain region for each pain stage by using the above.

More specifically, in the present disclosure, the pain template means that the availability of an indicator substance measured according to the intensity of pain in at least two or more brain regions of a reference individual is shown according to the intensity of pain and each brain region. For example, in the present disclosure, the pain template may measure the availability of mGluR5 in a specific region of the brain of a reference individual using [11C]ABP688, and shows the availability of the indicator substance for each specific brain region at the corresponding pain stage by using the above, but is not limited thereto.

In the present disclosure, the pain template may divide the pain level as at least 10 or more stages, 20 or more stages, 30 or more stages, 40 or more stages, 50 or more stages, 60 or more stages, 70 or more stages, 80 or more stages, 90 or more stages, 100 or more stages, 110 or more stages, 120 or more stages, 130 or more stages, 140 or more stages, 150 or more stages, 160 or more stages, 170 or more stages, 180 or more stages, 190 or more stages, or 200 or more stages. More preferably, it may divide the pain level as at least 100 or more stages. Still more preferably, it may be to divide the pain level as at least 200 or more stages.

In the present disclosure, the pain template may subdivide pain stages through regression analysis. For example, it may be subdivided into 200 pain stages by performing regression analysis of the availability of an indicator substance measured according to the intensity of pain in at least two brain regions of 10 reference individuals. The regression analysis may be performed using a least squares method, but is not limited thereto.

The method for measuring the intensity of pain in a test individual according to the present disclosure may include determining the intensity of pain in a test individual having an unknown pain intensity using a pain template obtained from the reference individual. Specifically, the determination of the pain intensity of the test individual is performed by applying the availability of the indicator substance measured in the at least two brain regions of the test individual to the pain template, analyzing the availability of the indicator substance and the correlation coefficient for each stage of the pain intensity, selecting a stage of the pain intensity having the highest correlation, and determining the stage of the pain intensity of the test individual. In the present disclosure, the pain intensity of the test individual may be measured through the step of comparing the availability of an indicator substance for each brain region of the test individual with the pain template.

Specifically, the availability of an indicator substance measured in at least two brain regions of the test individual is compared with each pain intensity in the pain template, and the intensity of the pain determined to have the highest correlation may be determined as the pain intensity of the test individual. The indicator material is preferably the same as the indicator material of the reference individual used in the step of generating the pain template.

Specifically, the step of determining the pain intensity of a test individual using a pain template includes (i) measuring the availability of the indicator substance measured in the at least two brain regions of the test individual, (ii) applying the measured availability of the indicator substance to the pain template to perform an analysis of the availability and similarity of the indicator substance for each stage of pain intensity, (iii) selecting the stage of the pain intensity having the highest similarity from the results of the similarity analysis to determine the stage of the pain intensity of the test individual.

For example, the availability of indicator substances for each brain region measured in the test individual and the availability of indicator substances for each brain region in each of the pain levels 1 to 200 of the pain template were calculated by calculating the degree of matching through similarity analysis, and as a result, when 100 stages of pain level and the availability of indicator substances for each brain region are found to be the most matching, it may mean determining the intensity of the pain of the test individual as 100 stages out of 1 to 200 stages.

In the similarity analysis, the similarity may be calculated through an algorithm for calculating a similarity degree, and for example, it may be performed by one or more methods selected from the group consisting of a method of calculating the degree of correlation using Pearson's correlation coefficient analysis, a method for calculating the degree of correlation using Spearman's correlation coefficient analysis, a method of calculating a similarity degree using each Euclidean distance on a multidimensional coordinate plane, a method of calculating a similarity degree using Mahalanobis distance, a method of calculating a similarity degree using a support vector, a method of calculating a similarity degree using the cosine distance, a method of calculating a similarity degree using Manhattan distance, a method of calculating a similarity degree using a Jaccard coefficient, and a method of calculating a similarity degree using extended Jaccard coefficient, but is not limited thereto.

For example, the degree of matching between the availability of the indicator substance for each brain region measured in the test individual and the availability of the indicator substance in each pain stage of the pain template may be calculated using the Pearson correlation coefficient analysis method. More specifically, the pain stage of the pain template in which the value of the correlation coefficient calculated by the following Mathamatical Formula 2 is closest to 1 may be determined as the intensity of the pain of the test individual.

The Pearson correlation analysis method is an analysis method used to find the correlation between two variables, and the Pearson correlation coefficient for two variables X and Y is the value obtained by dividing the degree to which X and Y change together by the degree to which X and Y change respectively. The Pearson correlation coefficient can be calculated by the following Mathematical Formula 1.

## [Mathematical Formula 1]】

$$r = \frac{\sum_{i=1}^{n}(x_i - \bar{x})(y_i - \bar{y})}{(n-1)(s_x s_y)}$$

wherein, $\bar{x}$ represents the sample mean for the variable X, $\bar{y}$ represents the sample mean for the variable Y, $s_x$ represents the standard deviation for the variable X, and $s_y$ represents the standard deviation for the variable Y. The Mathematical Formula 1 may be summarized as the following Mathematical Formula 2.

## [Mathematical Formula 2]

$$r = \frac{\sum_{i=1}^{n}(x_i - \bar{x})(y_i - \bar{y})}{\sqrt{\sum_{i=1}^{n}(x_i - \bar{x})^2 \sum_{i=1}^{n}(y_i - \bar{y})^2}}$$

in the Mathematical Formulae 1 and 2, the variable X is a standardized value obtained by dividing the availability of an indicator substance measured in n regions of interest (ROI) of the brain of the test individual by the mean value for each region.

In the Mathematical Formulae 1 and 2, the variable Y is the availability of an indicator substance in any one of the pain stages of the pain template.

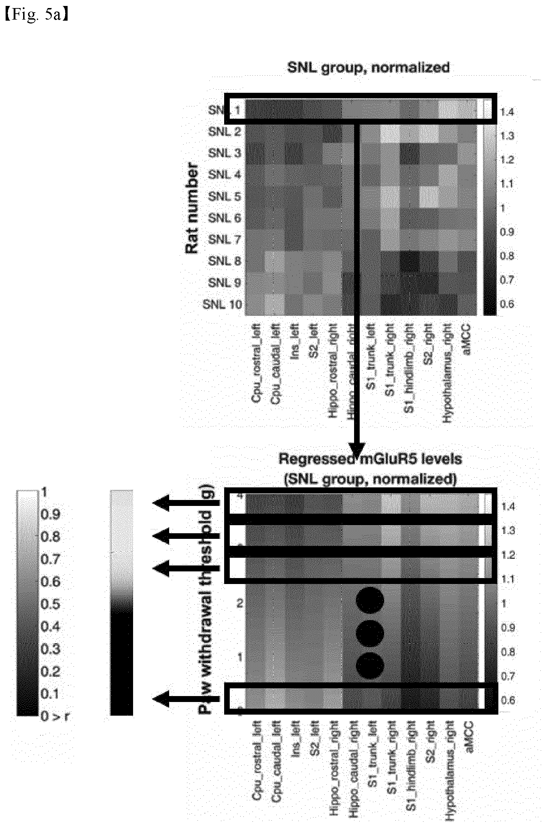

More specifically, the variable X is a standardized value obtained by measuring the availability of an indicator substance in n brain regions of the test individual, and then dividing it by the mean value of the availability of the indicator substance for each brain region already obtained from a plurality of control individuals. That is, the variable X is the standardized availability of indicator substances measured in n brain regions of the test individual, $\bar{x}$ is the mean value of standardized values for the availability of an indicator substance measured in n brain regions of a test individual. In other words, the variable X is the data of the test object, which is, for example, shown in a red square in the upper part in FIG. 5a shows an example of the variable X.

More specifically, the variable Y is the availability of an indicator substance measured in n brain regions at one stage of pain in the pain template. That is, the variable Y is the availability of indicator substances in each of the n brain regions of any one pain stage in the pain template. For example, if the pain template is subdivided into pain intensity of 1 to 200 stages, it means the availability of an indicator substance for each brain region at any one stage of pain intensity. More specifically, when the paw withdrawal threshold ranging from 0 to 4 g, that is, pain intensity is subdivided into 200 stags to generate a pain template so as to have a range of 0.02g per stage, the variable Y represents the value of indicator substances in the n brain regions in the range of 0.02 g in any one stage of the pain templates. In other words, the variable Y is a value extracted from any one of the stages included in the pain template generated from the reference individual. For example, the lower part of FIG. 5a shows a plurality of black squares, showing an example of the variable Y.

Referring to FIG. 5a, it can be seen that the task of calculating whether the data (variable X) of one test individual has a certain degree of correlation coefficient with the value (variable Y) of one stage of the pain template is repeatedly calculated for each of 200 stages, and the results are shown.

Another embodiment of the present disclosure relates to a pain template, representing a correlation between the availability of an indicator substance measured in at least two brain regions of a reference individual and the intensity of pain induced in the reference individual.

The pain intensity may be classified into at least 10 or more stages, 50 or more stages, 100 or more stages, or 200 or more stages

Another embodiment of the present disclosure relates to a method for generating a pain template comprising the steps of: inducing artificial pain in a reference individual; measuring an availability level of an indicator substance in at least two brain regions of the reference individual in which the pain has been induced; and generating a pain template indicating a correlation between the intensity of pain artificially induced in the reference individual and the availability level of the indicator substance for each brain region.

The method of generating a pain template may further include the steps of dividing the measured availability level of the indicator substance for each brain region of at least two or more by the mean value of the availability level of the indicator substance for each brain region obtained in a control group in which pain has not been induced, to standardizing it; and subdividing pain intensity using the standardized values.

Another embodiment of the present disclosure relates to a method for generating a pain template comprising the steps of: inducing artificial pain in a reference individual, and selecting a brain region and an indicator substance having significance for pain; measuring an availability level of an indicator substance in at least two selected brain regions of the reference individual with the induced pain; measuring the availability level of the indicator substance in a control group with no induced pain, in a brain region corresponding to each of the brain regions in which the degree of availability of the indicator substance was measured in the reference individual with induced pain; dividing the availability level of the indicator substance for each brain region of the reference individual by the availability level of the indicator substance for each brain region of the control group corresponding thereto to standardize it; and divide the pain intensity into two or more stages using the value of the standardized availability level, and obtaining a pain template in which the availability level of the indicator substance is patternd for corresponding to each stage of the divided pain intensity.

The availability level of the indicator substance for each brain region of the control group can be a mean value of the availability level of the indicator substance for each brain region measured in at least two or more control groups.

Another embodiment of the present disclosure relates to a method of measuring the pain intensity of a test individual comprising comparing the availability of an indicator substance measured in at least two brain regions of the test individual with each pain stage of the pain template.

Yet another embodiment of the present disclosure relates to a method of measuring the pain intensity of the test individual comprising the steps of: inducing artificial pain in a reference individual, and selecting a brain region and an indicator substance having significance for pain; measuring an availability level of an indicator substance in at least two selected brain regions of the reference individual with the induced pain; measuring an availability level of an indicator substance in a control group with no induced pain, in a brain corresponding to each of the brain regions in which the availability level of the indicator substance is measured in a reference individual with induced pain; dividing the availability level of the indicator substance for each brain region of the reference individual by the availability level of the indicator substance for each brain region of the control group corresponding thereto to standardize it; dividing the pain intensity into two or more stages using the value of the standardized availability level, and obtaining a pain template in which the availability level of an indicator substance is patterned for corresponding to each stage of the divided pain intensity; measuring and standardizing an availability level of the indicator substance in the brain regions of the test individual, respectively, corresponding to the brain regions in which the availability level of the indicator substance in the reference individual is measured using the selected brain region and the indicator substance; comparing the measured availability level of the indicator substance of the test individual through similarity analysis, with the availability level of the indicator substance for each stage of the pain intensity in the pain template; and determining a stage of pain intensity in which an availability level of an indicator substance has the highest similarity in a pain template in the comparing step, as a stage of pain intensity of the test individual.

The availability level of the indicator substance for each brain region of the control group may be a mean value of the availability level of the indicator substance for each brain region measured in at least two or more control groups.

The comparing step may be performed through similarity analysis.

The method for measuring the pain intensity of the test individual may further include a step of determining the stage of the pain intensity in the pain template having the highest similarity in the comparing step as the stage of the pain intensity of the test individual.

**[ADVANTAGEOUS EFFECTS]**

When a pain template is generated as a comparison criterion according to an embodiment of the present disclosure, it is sufficient to measure only the expression pattern of the indicator substance in the test individual for the diagnosis of pain in a test individual with unknown pain intensity, and linguistic communication with the test individual, physical diagnosis, or behavioral measurements are not required to determine the pain level. There is no need to deliberately induce pain by applying external stimuli to the test individual.

**[BRIEF DESCRIPTION OF THE DRAWINGS]**

FIG. 1a shows the distribution of the paw withdrawal thresholds measured using von Frey filament at 15 days after SNL surgery was performed on 103 mice.
FIG. 1b shows the distribution of the paw withdrawal threshold of 10 selected mice in which neuropathic pain was successfully induced.
FIGS. 2a to 2d illustrate regions showing negative interrelation with the paw withdrawal threshold among the regions showing significance involved in pain among the brain regions.
FIGS. 3a to 3f illustrate regions showing positive interrelation with the paw withdrawal threshold among regions showing significance related to pain among brain regions.
FIG. 4a shows mGluR5 values for each brain region of SNL individuals.
FIG. 4b shows mGluR5 values for each brain region of Sham surgery group individuals.
FIG. 4c shows that the standardization is performed by dividing mGluR5 values for each brain region of SNL individuals by the mean value for each region.
FIG. 4d shows that standardization is performed by dividing mGluR5 values for each brain region of the individuals in the Sham surgery group by dividing them the mean value for each region.
FIG. 4e shows a pain template showing intracerebral mGluR5 patterns of the pain group according to the pain level.
FIG. 5a shows the task of comparing intracerebral mGluR5 information of SNL 1 with a pain template.
FIG. 5b shows the process of calculating the correlation coefficient of the pattern of each experimental animal in the pain group for the pain template.
FIG. 6a shows the pattern of mGluR5 values of SNL individuals and the r-value of the pattern of pain templates.
FIG. 6b shows the p-value of the pattern of mGluR5 values of SNL individuals and the pattern of the pain template.
FIG. 6c shows the inverse estimation of the original paw withdrawal threshold of the experimental animal through the high degree of correlation coefficient.
FIG. 6d shows the pattern of mGluR5 values of Sham individuals and the r-value of the pattern of pain templates.
FIG. 6e shows the pattern of mGluR5 values of Sham individuals and the p-value of the pattern of pain templates.
FIG. 6f is a graph showing sensitivity and specificity according to r-value.

**[DETAILED DESCRIPTION OF THE EMBODIMENTS]**

Hereinafter, the present disclosure will be described in more detail by way of examples. However, the present disclosure is for illustrative purposes only and the scope of the present disclosure is not limited thereto.

**Example 1: Preparation of a reference individual through the construction of a pain model**

To measure the pain level in a patient with neuropathic pain or laboratory animal (hereinafter referred to as "individual"), the paw withdrawal threshold to von Frey filament stimulus was measured. Individuals who have induced pain in spinal nerve ligation (hereinafter referred to as "SNL") surgery have various pain levels, depending on the individual.
8-week-old male Sprague-Dawley rats (Samtako, Seoul) were anesthetized with isoflurane and subjected to right L5 spinal nerve ligation (SNL), and the control group underwent sham surgery.
In the SNL surgical group, the right L5 spinal nerve was isolated and tightly ligated using 5-0 silk to induce neuropathic pain. In the shame group as a control group, L5 spinal nerves were isolated but not ligated.
The paw withdrawal thresholds on the right hind leg that performed surgery immediately before surgery and at 1, 5, 9, and 15 days after surgery were measured using von Frey filaments. Animals with abnormal motor neuropathy after surgery were excluded from the analysis.

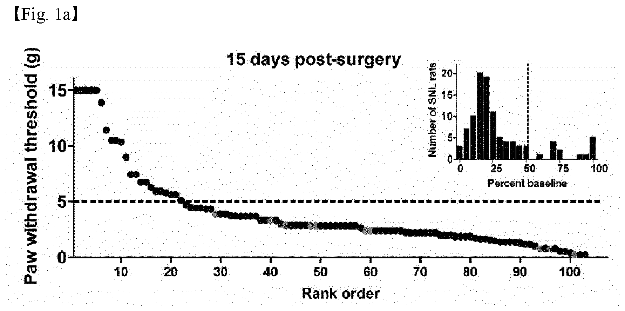

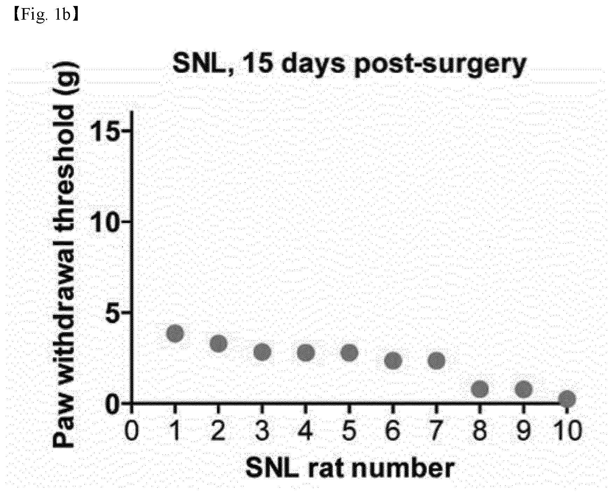

FIG. 1a shows the distribution of paw withdrawal thresholds measured using von Frey filaments at 15 days after SNL surgery was performed on 103 mice. As the mouse's paw withdrawal threshold (y-axis in FIG. 1a) is smaller, it is sensitive even to a smaller stimulus. It was judged that neuropathic pain was successfully induced when the paw withdrawal threshold was reduced to less than half compared to before surgery. 10 of the mice in which neuropathic pain was successfully induced were selected, and the selected mice are indicated in red in FIG. 1a. The paw withdrawal threshold of the selected mice was shown in FIG. 1b, and these mice were subjected to PET scan. FIG. 1b shows the distribution of the paw withdrawal thresholds of 10 selected among the mice in which neuropathic pain was successfully induced.

**Example 2: Measurement of indicator substance**

Metabotropic glutamate receptor 5 (hereinafter, referred to as "mGluR5") in the brain of the pain model of the reference individual was measured. To measure this, [11C]ABP688, a tracer with radioactive isotope [11C] attached to ABP688, a chemical substance that specifically binds to mGluR5, was used. The signal of the PET image can be converted into non-displaceable binding potential information using the Simplified reference tissue model, and this information indicates the availability of mGluR5 in each coordinate space. Specifically, the pain model was anesthetized with isoflurane, and [11C] ABP688 (5.05-16.15 MBq / 100g) was injected into a tail vein. Brain images were obtained using a micro-PET/CT scanner (eXplore VISTA, GE Healthcare) in list-mode for 60 minutes. The mGluR5 binding potential (non-displaceable binding potential, BPND) of [11C] ABP688 was calculated using a simplified reference tissue model with the cerebellum as a reference region. All [11C] ABP688 BPND images were averaged to create a brain mGluR5 standard image, and then all BPND images were spatially normalized to brain mGluR5 standard images. The 3D pixels were resampled to 0.2 * 0.2 * 0.2 mm, and smoothed with a 0.8 mm full-width at half maximum Gaussian filter. Images were processed using SPM8, MarsBaR tool box and Turku PET Center's imgsrtm program.

**Example 3: Generating a Pain Template**

3-1: Searching for brain regions involved in pain

Based on the experimental data on the pain level measured in Examples 1 and 2, regression analysis was performed on the intracerebral mGluR5 availability information obtained with [11C]ABP688-PET.

Specifically, to search for the correlation between the intracerebral mGluR5 level and the paw withdrawal threshold, 3D pixel (voxel) regression analysis was performed using data from SNL group animals. Through regression analysis, clusters of more than 20 voxels having a statistically significant (p-value<0.005) correlation with the paw withdrawal threshold were screened. Based on the anatomical position of the cluster, each sphere-shaped region with a radius of 0.5 mm was set as a region-of-interest (ROI), and BPND was extracted from each ROI using MarsBaR toolbox.

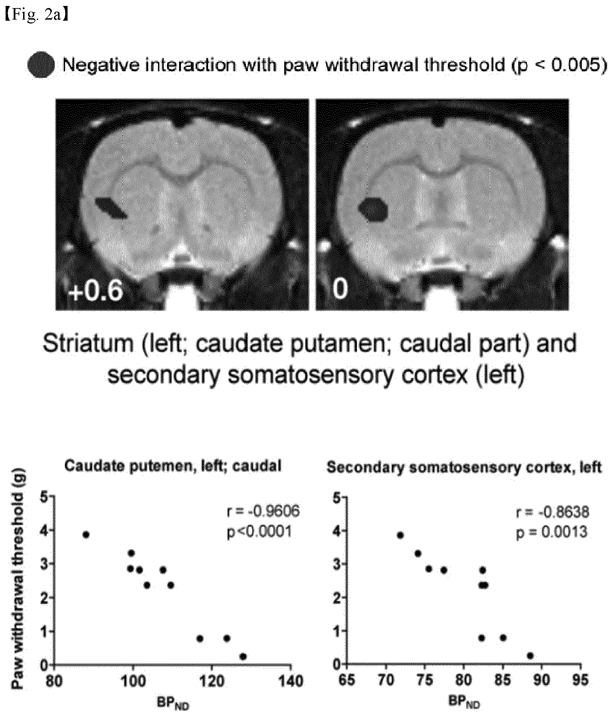

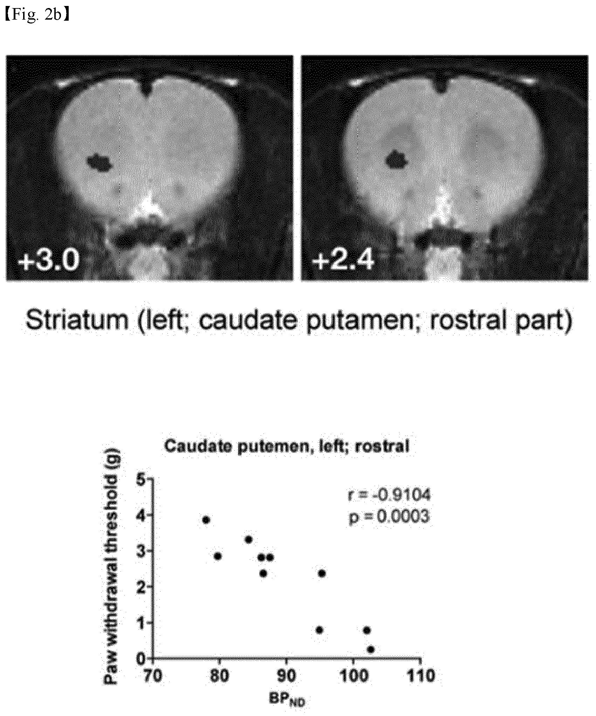

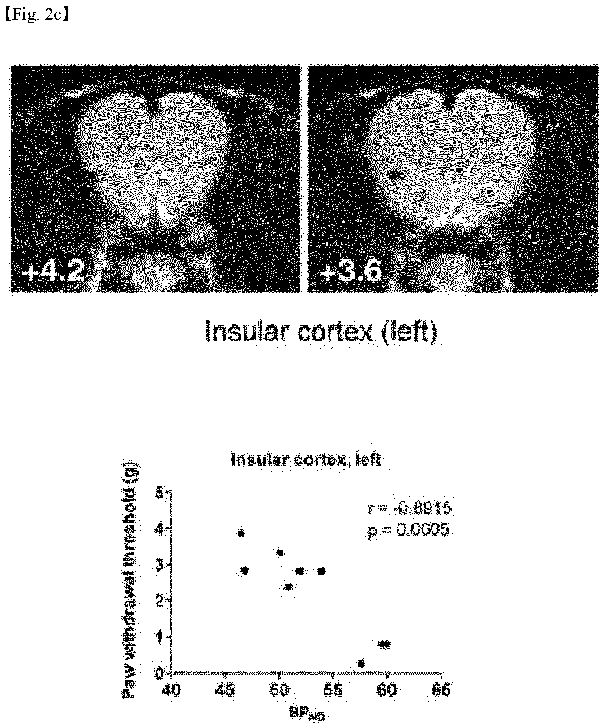

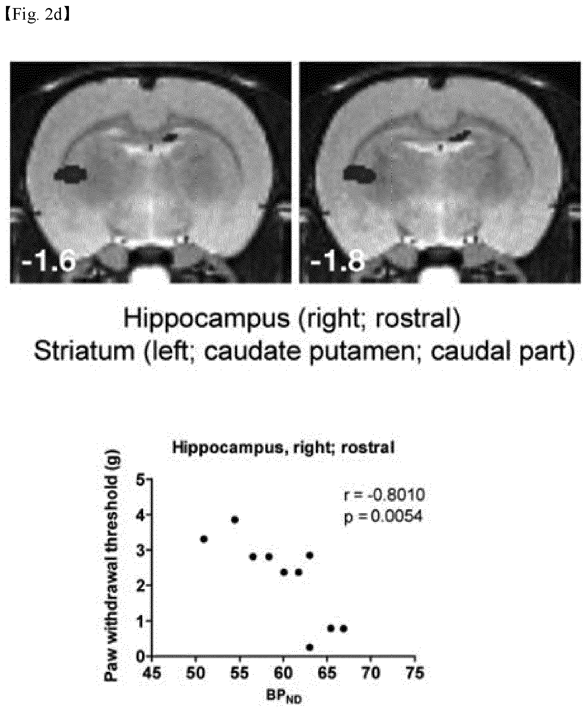

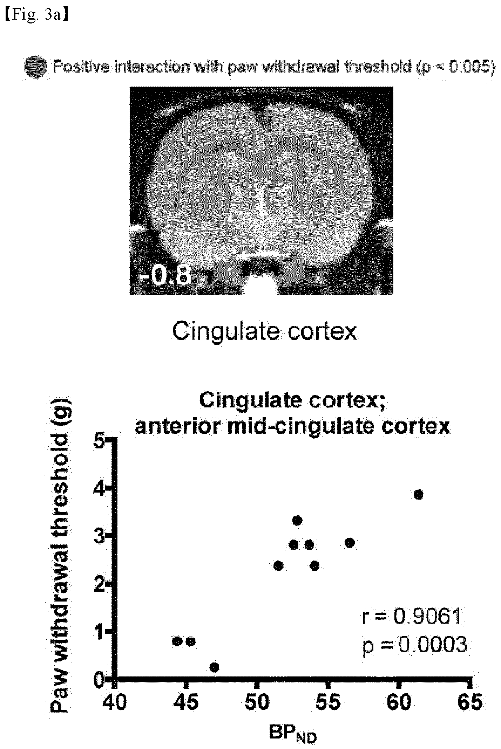

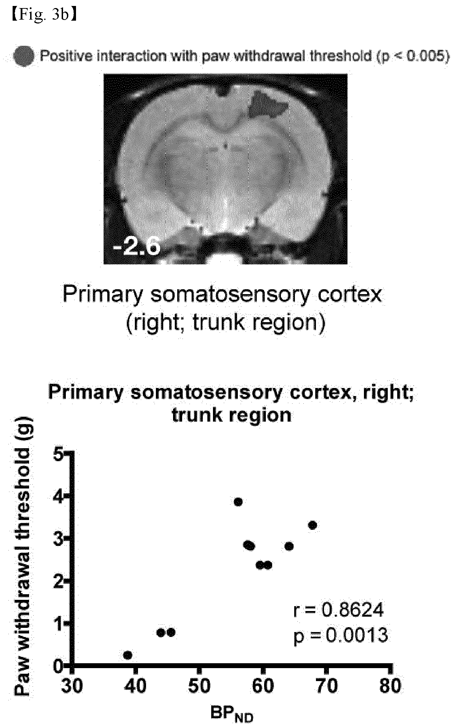

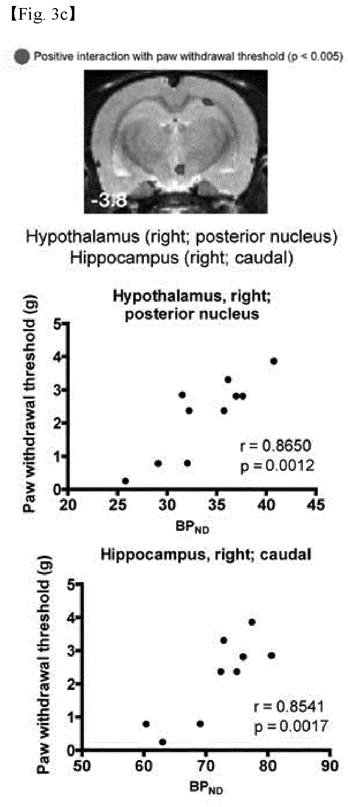

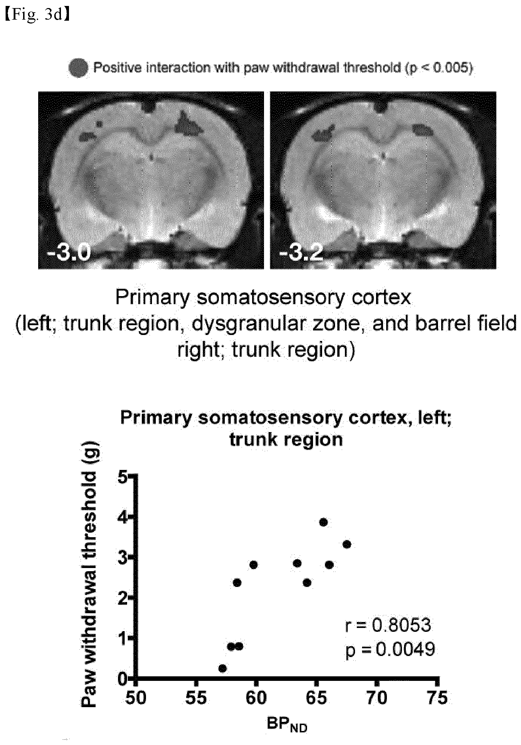

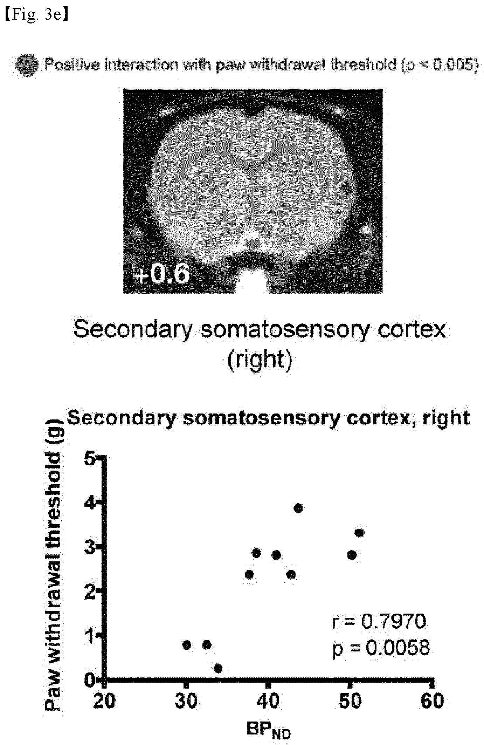

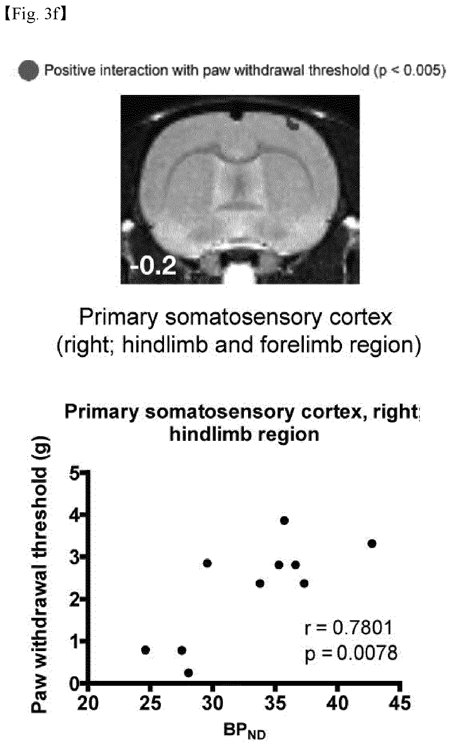

As a result, significant correlations were found in several brain regions involved in pain. In order to visualize brain regions showing significance, the regions were superimposed on MRI images and shown in FIGS. 2a to 2d and FIGS. 3a to 3f.

FIGS. 2a to 2d are regions showing negative interaction with the paw withdrawal threshold, and FIGS. 3a to 3f are regions showing positive interaction with the paw withdrawal threshold. In the graph below the image of each brain region, the mGluR5 value in the ROI (Region of Interest) was shown on the x-axis, and the paw withdrawal threshold (representing the degree of pain in the experimental animal) was shown on the y-axis.

3-2: Determination of pain template

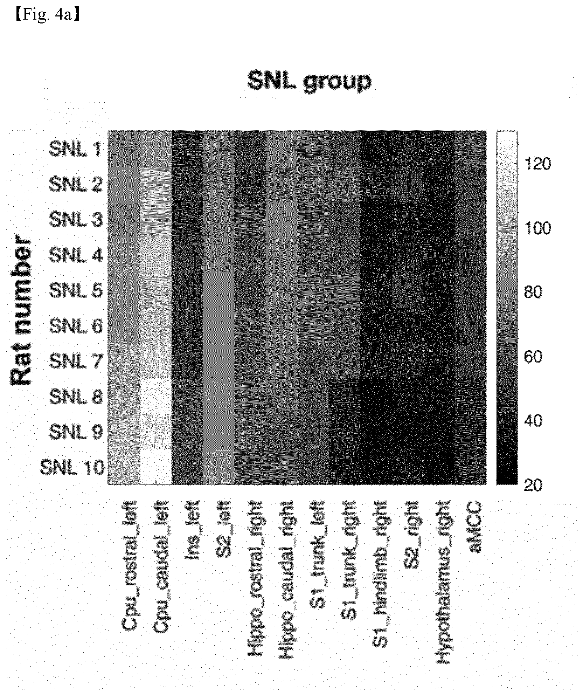

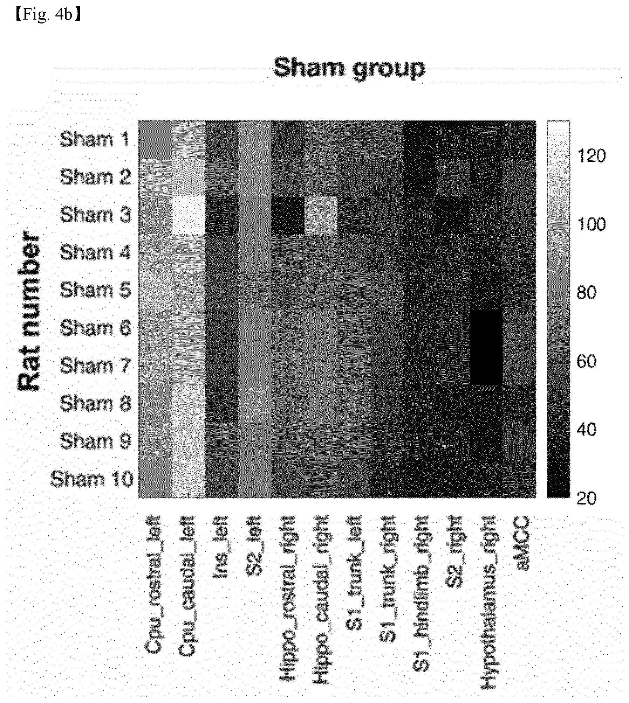

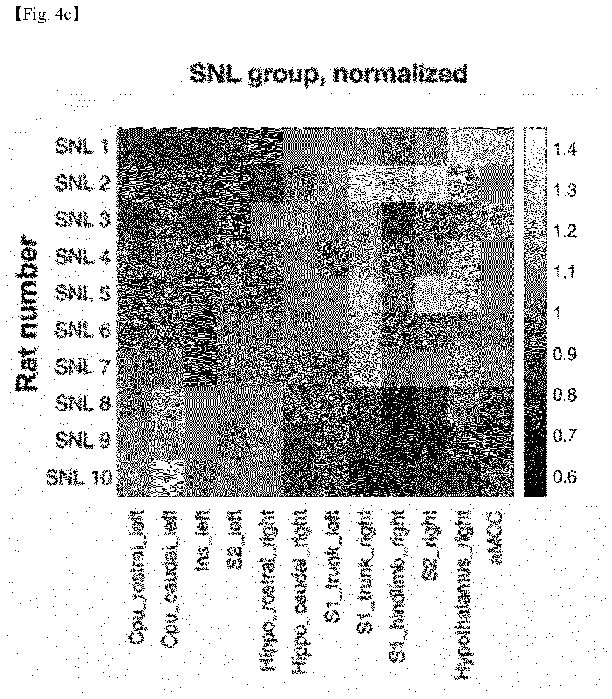

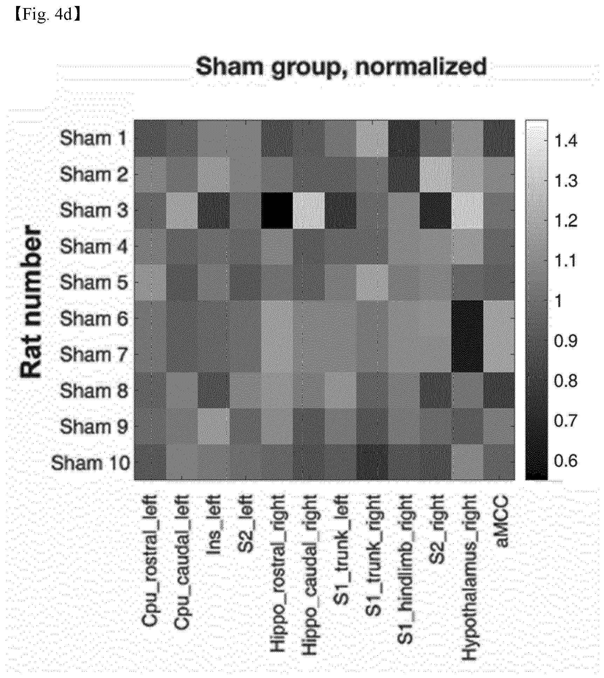

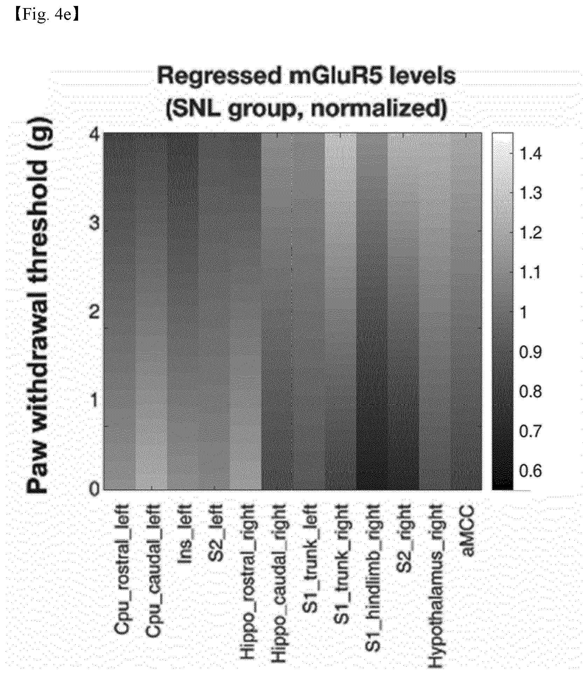

An area of the same size was defined for each coordinate, and mGluR5 values were extracted and shown for each individual in FIG. 4. In FIG. 4, SNL 1 to SNL 10 are identification numbers of each experimental animal, SNL 1 has a high paw withdrawal threshold, and SNL 10 has a low paw withdrawal threshold. That is, it can be seen that SNL 1 has the relatively weaker degree of insensitive pain among the 10 pain groups, and SNL 10 is the most sensitive and the degree of pain is most severe.

The y-axis represents each experimental animal individual of SNL 1 to SNL 10, and the x-axis represents brain regions that were statistically significantly correlated with the paw withdrawal threshold in the above analysis. The mGluR5 values in each region had different distributions in each brain region (FIG. 4a). Thus, the standardization was performed by dividing the mGluR5 values of each area of the individuals by the mean value for each area, and shown in FIG. 4c. The mean value for each area was calculated based on data obtained from the control group (Sham surgery group) without pain. The normalized mGluR5 levels in each brain region were regressed for the paw withdrawal threshold.

More specifically, if n paw withdrawal thresholds obtained from n reference individuals are taken as the independent variable x, and n of mGluR5 values extracted from any one ROI and standardized are set as the dependent variable y, the mGluR5 value of the region of interest can be represented by the following Mathematical Formula 3.

[Mathematical Formula 3]

$$y = \beta_0 + \beta_1 x + \varepsilon$$

in the Mathematical Formula 3, $\beta_0$ is the y-intercept, $\beta_1$ is the regression coefficient (the slope of the linear equation), and $\varepsilon$ is the error. The relational expression of $y = \beta_0 + \beta_1 x$ was obtained through regression analysis, and the mGluR5 value in the region of interest was predicted therefrom.

First, a regression analysis using the least squares method was performed as follows, and the regression coefficient $\beta_1$ was estimated.

[Mathematical Formula 4]

$$\beta_1 = \frac{\sum_{i=1}^{n}(x_i - \bar{x})(y_i - \bar{y})}{\sum_{i=1}^{n}(x_i - \bar{x})^2}$$

wherein, $\bar{x}$ represents the mean value of the independent variable x (paw withdrawal threshold), and y represents the mean value of the dependent variable y (mGluR5 value extracted from one region of interest and standardized).

Based on the above results, the y-intercept $\beta_0$ was estimated.

[Mathematical Formula 5]

$$\beta_0 = \bar{y} - \beta_1 \bar{x}$$

The mGluR5 value of the region of interest was estimated using the obtained relational expression y = $\beta_0 + \beta_1 x$ and displayed in one column.

The regression analysis as described above was individually performed in each region of interest, and then the estimated values obtained in each region were shown in individual columns (FIG. 4e).

As shown in FIG. 4e, through the above regression analysis, the pain stage in the range of 0 to 4 g of the paw withdrawal threshold was divided into 200 stages, and the mGluR5 availability in each ROI obtained through the regression analysis was displayed in 200 rows in each column. As such, the rows of the regressed mGluR5 template represent the ideal mGluR5 pattern of a virtual SNL individual with a corresponding paw withdrawal threshold. This makes it possible to estimate the intracerebral mGluR5 pattern possessed by the virtual individual with the corresponding paw withdrawal threshold (pain level) (Fig. 4e). That is, the figure shown in FIG. 4e is calculated based on FIG. 4c, the intracerebral mGluR5 patterns of the pain group are shown according to the pain level, and will be a reference for comparison to determine the presence or absence of neuropathic pain and the degree of pain in the future.

This comparison criterion will be referred to as "pain template" in the future. The data of the control group without pain are shown in FIGS. 4b and 4d. In the control group, no pattern visible from the pain group could be seen.

**Example 4: Objective Measurement of Pain Using Pain Template**

4-1: Verification of pain template

When the pain template was used and the intracerebral mGluR5 information was given, by comparing which part of the pain template this information matches, the presence or absence of pain and the pain level can be determined. For example, the task of comparing intracerebral mGluR5 information of experimental animal No. 1 (SNL 1) within the pain group with a pain template is shown in FIG. 5a.

Since the pain template was estimated based on the actual information of the experiment animals 1 to 10, the pattern of SNL 1 would match with the pattern in the top row of the pain template. Whether each line of the SNL 1 pattern and the pain template also showed a correlation to some extent was compared. SNL 1 had a paw withdrawal threshold of 3.86 g. FIG. 5a shows a procedure of calculating Pearson's correlation coefficient and displaying it in red as the correlation coefficient increases. The row indicated by the red square is the mGluR5 pattern of SNL 1, and this row was compared one by one with each row of the pain template shown at the bottom. The correlation coefficient for each row is indicated in color at the lower left.

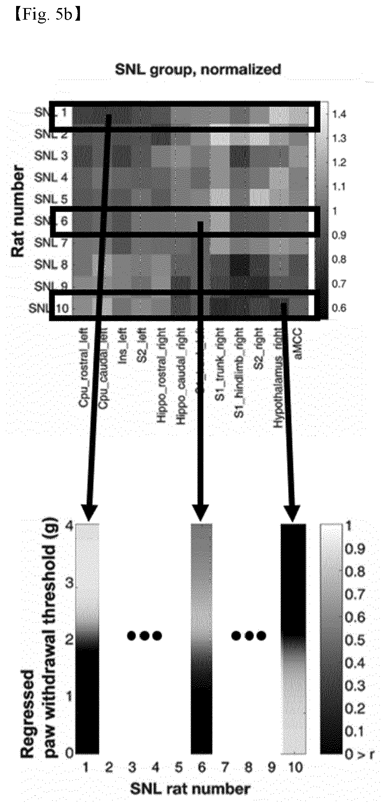

This operation was repeated for each experimental animal (SNL 1 to SNL 10), and whether each experimental animal matches with each row of the pain template to some extent can be represented by a graph. The process of calculating the correlation coefficient that each laboratory animal pattern has for the pain template is illustrated in FIG. 5b.

4-2: Objective measurement of pain using a pain template

Through the same process as in Example 4-1, it is possible to calculate how much the pattern of the standardized mGluR5 value of each individual is similar to the pattern (pain template) of the reference individual. There are several methods that can calculate this, but here, the similarity was calculated through Pearson's correlation coefficient analysis method.

The Pearson correlation analysis method is an analysis method used to find the correlation between two variables. The Pearson correlation coefficient of the two variables X and Y is the value obtained by dividing the degree to which X and Y change together, by the degree to which X and Y change respectively. The method for calculating the sample correlation coefficient is as follows.

[Mathematical Formula 1]

$$r = \frac{\sum_{i=1}^{n}(x_i - \bar{x})(y_i - \bar{y})}{(n-1)(s_x s_y)}$$

wherein, $\bar{x}$ represents the sample mean for the variable X, $\bar{y}$ represents the sample mean for the variable Y, $s_x$ represents the standard deviation for the variable X, and $s_y$ represents the standard deviation for the variable Y. The above Mathematical formula may be summarized as follows.

[Mathematical Formula 2]

$$r = \frac{\sum_{i=1}^{n}(x_i - \bar{x})(y_i - \bar{y})}{\sqrt{\sum_{i=1}^{n}(x_i - \bar{x})^2 \sum_{i=1}^{n}(y_i - \bar{y})^2}}$$

in the Mathematical Formulae 1 and 2, the variable X is a standardized value obtained by dividing the availability of an indicator substance measured in n regions of interest (ROI) of the brain of the test individual by the mean value for each region. More specifically, the variable X is a standardized value obtained by measuring the availability of an indicator substance in n brain regions of the test individual, and then dividing it by the mean value of the availability of the indicator substance for each region already obtained from a plurality of control individuals. That is, the variable X is the standardized availability of indicator substances measured in n brain regions of the test individual, $\bar{x}$ is the mean value of standardized values for the availability of an indicator substance measured in n brain regions of a test individual. In other words, the variable X is the data of the test object, which is, for example, shown in a red square in the upper part in FIG. 5A shows an example of the variable X.

In the Mathematical Formulae 1 and 2, the variable Y is the availability of an indicator substance in any one of the pain stages of the pain template. More specifically, the variable Y is the availability of an indicator substance measured in n brain regions at one stage of pain in the pain template. That is, the variable Y is the availability of indicator substances in each of the n brain regions in any one pain stage in the pain template. For example, if the pain template is subdivided into pain intensity of 1 to 200 stages, it means the availability of an indicator substance for each brain region at any one stage of pain intensity. More specifically, when the paw withdrawal threshold ranging from 0 to 4, that is, pain intensity is subdivided into 200 steps to generate a pain template so as to have a range of 0.02g per stage, the variable Y represents the value of indicator substances in the n brain regions in the range of 0.02 g for any one of the pain templates. In other words, the variable Y is a value extracted from any one of the stages included in the pain template generated from the reference individual, for example, the lower part of FIG. 5A shows a plurality of black squares, showing an example of the variable Y.

Here, let's assume that the data of an arbitrary individual (test individual) actually observed is assigned to the variable X, and data from one of the 200 rows in the pain template is assigned to the variable Y. By comparing the correlation coefficients of these two variables, the pattern of mGluR5 availability in the brain extracted from n ROIs in a given individual can be compared with the pattern in one row of the corresponding pain template. The value of the Pearson correlation coefficient r has a value between -1 and 1. As the correlation coefficient r is closer to 1, the two variables can have a stronger amount of positive correlation (i.e., the higher the similarity). By repeating this analysis for 200 rows of the pain template, it is possible to calculate whether the mGluR5 expression pattern possessed by any individual is most similar to any row of the pain template. Referring to FIG. 5a, it can be seen that the task of calculating how the data (variable X) of one test individual has a correlation coefficient with the value of the pain template stage (variable Y) to some extent is repeatedly calculated for each of 200 stages, and the result is displayed. Here, since a pain template with a range of 0 to 4 g of paw withdrawal threshold divided by 200 rows was created and used, one row of the pain template has a range of 0.02 g. The r value that an individual has for each 200 rows of the pain template is represented by one column, and the upper 25% r-value range in one column was taken to estimate the paw withdrawal threshold within the range of 1 g.

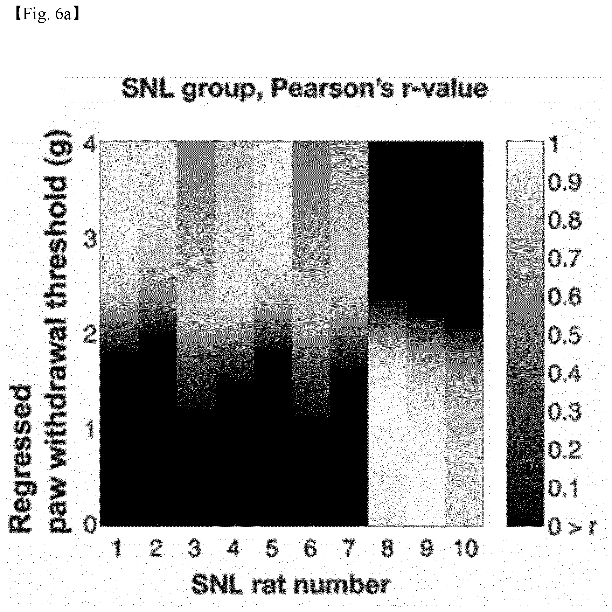

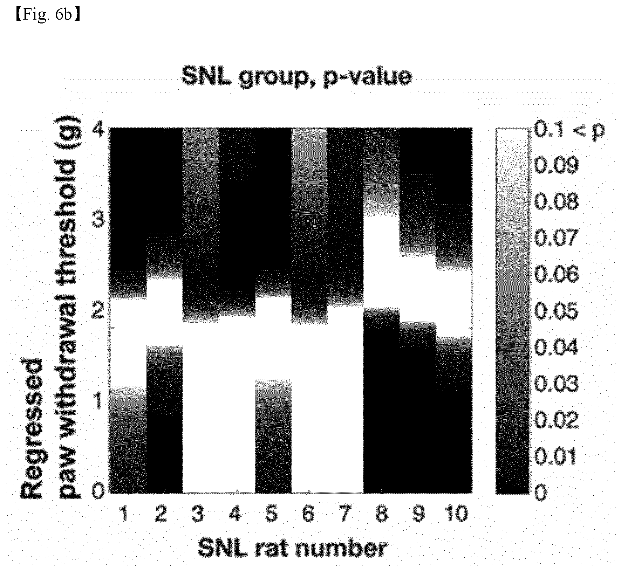

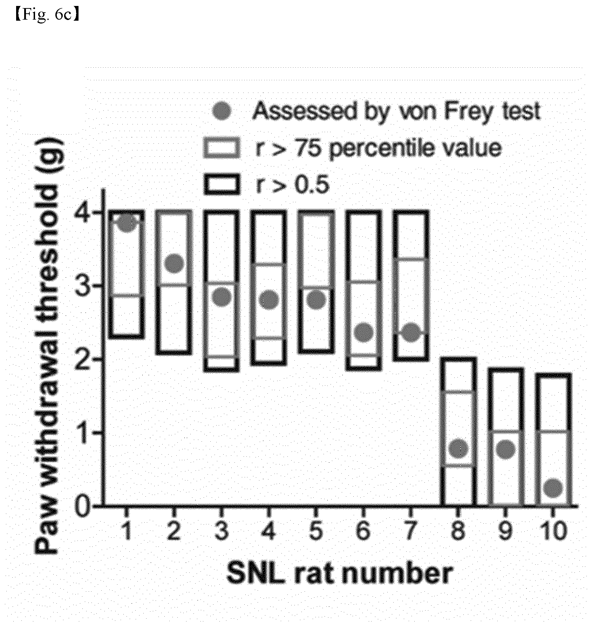

When comparing the patterns of 10 individuals in the SNL group to 200 rows of each pain template by correlation coefficient analysis, it was confirmed that all individuals well matched with the pattern of certain rows present in the pain template, which was well confirmed by the r value and p value of the correlation coefficient (FIGS. 6a and 6b). In addition, through the high degree of correlation coefficient, it was possible to successfully back-estimate the original paw withdrawal threshold of the experimental animal (FIG. 6c).

4-3: Comparison with the control group

In order to confirm whether this method can successfully distinguish only the pain group, this time, the mGluR5 pattern in the brain of the Sham group without pain was compared with the pattern of the pain template.

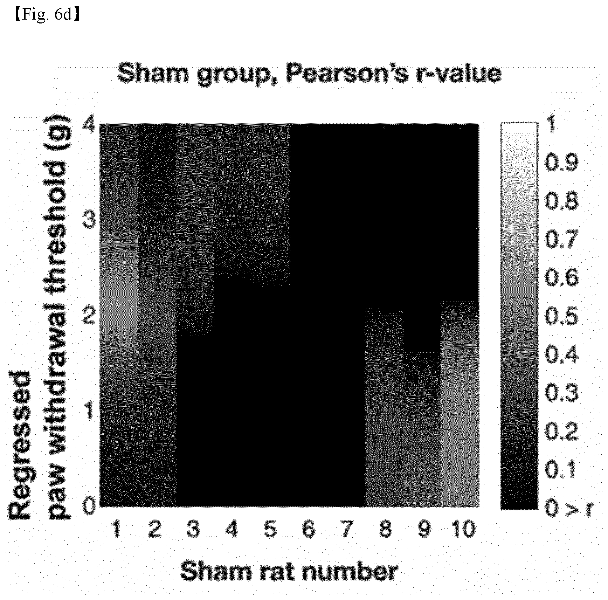

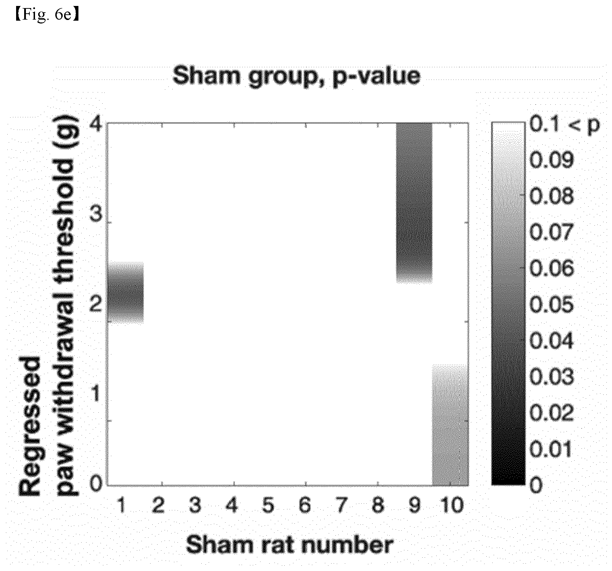

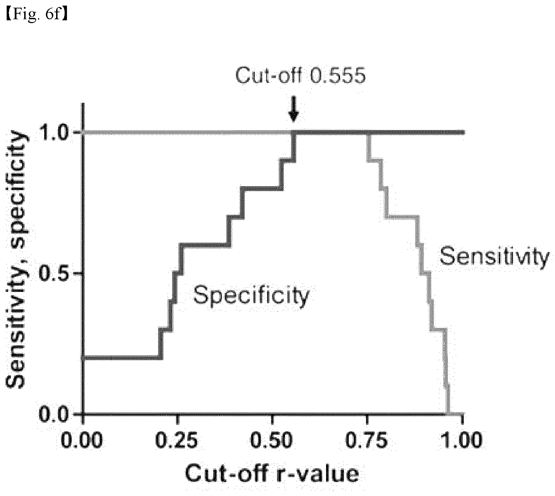

As a result, the pattern of the control group did not match well with the pain template, unlike the pain group. The r-value of the correlation coefficient was generally low. The p value of the correlation coefficient also did not guarantee statistical significance (FIGS. 6d, 6e and 6f). The presence or absence of pain could be predicted by classifying the criteria of the degree of matching through the correlation coefficient (FIG. 6f).

**Claims**

1. A method for measuring pain intensity comprising the steps of:

   generating a pain template that indicates a correlation between availabilities of an indicator substance measured at each pain intensity in at least two or more brain regions of a reference individual, and each brain region and each stage of pain intensity; and
   applying availabilities of the indicator substance measured in the at least two brain regions of a test individual to the pain template, and selecting the stage of the pain intensity having the highest similarity with the availability of the indicator substance for each stage of the pain intensity through similarity analysis, to determine the pain intensity of the test individual.

2. The method according to claim 1, wherein the at least two or more brain regions are regions in which the availability of the indicator substance changes due to pain.

3. The method according to claim 1, wherein the at least two or more brain regions are two more selected from the group consisting of: (1) rostral caudate-putamen of striatum, left, (2) caudal caudate-putamen of striatum, left, (3) insular cortex, left, (4) secondary somatosensory cortex, left, (5) hippocampus, right; rostral, (6) hippocampus, right; caudal, (7) primary somatosensory cortex, left; trunk region, (8) primary somatosensory cortex, right; trunk region, (9) primary somatosensory cortex, right; hindlimb region, (10) secondary somatosensory cortex, right, (11) hypothalamus, right; posterior nucleus, and (12) anterior midcingulate cortex.

4. The method according to claim 1, wherein the pain template is generated by performing a step of standardizing with dividing the availability of indicator substances for at least two or more brain regions by a mean value of the availability of the indicator substance for each brain region; and a step of subdividing the pain intensity through regression analysis of the standardized value, and wherein the mean value of the availability of the indicator substance for each brain region is the mean value of the availability of the indicator substance for each brain region obtained from a painless control group.

5. The method according to claim 1, wherein the similarity analysis is performed by one or more analysis methods selected from the group consisting of Pearson's correlation coefficient analysis, Spearman's correlation coefficient analysis, Euclidean distance analysis, Mahalanobis distance analysis, Support vector analysis, Cosine distance analysis, Manhattan distance analysis, Jaccard coefficient analysis, and Extended Jaccard coefficient analysis.

6. The method according to claim 5, wherein the Pearson's correlation coefficient analysis is performed by the following Mathematical Formula 2, and the similarity is evaluated to an extent where the r value calculated by Mathematical Formula 2 is close to 1.

[Mathematical Formula 2]

$$\frac{\sum_{i=1}^{n}(x_i - \bar{x})(y_i - \bar{y})}{\sqrt{\sum_{i=1}^{n}(x_i - \bar{x})^2 \sum_{i=1}^{n}(y_i - \bar{y})^2}}$$

X: standardized availability of indicator substances measured in brain regions of test individuals
Y: availability of indicator substances possessed by any one pain stage of the pain template
$\bar{x}$: sample mean of X
$\bar{y}$: sample mean of Y
n: number of brain regions

7. The method according to claim 1, wherein the indicator substance is metabotropic glutamate receptor 5 (mGluR5).

8. The method according to claim 1, wherein the availability of the indicator substance is measured by using a radioactive isotope tracer specific to the indicator substance.

9. The method according to claim 8, wherein the radioactive isotope tracer is [11C]ABP688.

10. The method according to claim 1, wherein the availability of the indicator substance measured according to the intensity of pain is obtained by using the reference individual of at least 10 or more individuals.

11. The method according to claim 1, wherein the reference individual has induced neuropathic pain in a right hind leg.

12. A method for generating a pain template comprising the steps of:

inducing artificial pain in a reference individual, and selecting a brain region and an indicator having significance for pain;
measuring an availability level of an indicator substance in at least two or more selected brain regions of the reference individual with the induced pain;
measuring the availability level of the indicator substance in a control group with no induced pain, in a brain region corresponding to each of the brain regions in which the degree of availability of the indicator substance was measured in the reference individual with the induced pain;
standardizing the availability level of the indicator substance for each brain region of the reference individual through dividing by the availability level of the indicator substance for each brain region of the control group corresponding thereto; and
dividing the pain intensity into two or more stages using the value of the standardized availability level, and obtaining a pain template in which the availability level of the indicator substance is patterned for corresponding to each stage of the divided pain intensity.

13. A method for measuring a pain intensity of a test individual comprising the steps of:

inducing artificial pain in a reference individual, and selecting a brain region and an indicator substance having significance for pain; measuring an availability level of an indicator substance in at least two or more selected brain regions of the reference individual with the induced pain; measuring an availability level of an indicator substance in a control group with no induced pain, in a brain region corresponding to each of the brain regions in which the availability level of the indicator substance is measured in a reference individual with the induced pain; standardizing the availability level of the indicator substance for each brain region of the reference individual through dividing by the availability level of the indicator substance for each brain region of the control group corresponding thereto; dividing the pain intensity into two or more stages using the value of the standardized availability level, and obtaining a pain template in which the availability level of an indicator substance is patterned for corresponding to each stage of the divided pain intensity, measuring and standardizing an availability level of the indicator substance in the brain regions of a test individual, corresponding to the brain regions in which the availability level of the indicator substance in the reference individual is measured, by using the selected brain region and the indicator substance; comparing the measured availability level of the indicator substance of the test individual, with the availability level of the indicator substance for each stage of the pain intensity in the pain template through similarity analysis; and determining a stage of pain intensity in which an availability level of an indicator substance has the highest similarity in the pain template in the comparing step, as a stage of pain intensity of the test individual.

14. The method according to claim 12 or 13, wherein the availability level of the indicator substance for each brain region of the control group is a mean value of the availability level of the indicator substance for each brain region measured in at least two or more control groups.

15. The method according to claim 12 or 13, wherein the reference individual or the test individual is one or more selected from the group consisting of rodents, mice, rats, hamsters, guinea pigs, reptiles, amphibians, mammals, canines, felines, rabbit necks, pigs, cattle, sheep, monkeys, primates, non-human mammals, non-human primates, and humans.

【Fig. 1a】

【Fig. 1b】

SNL, 15 days post-surgery

【Fig. 2a】

● Negative interaction with paw withdrawal threshold (p < 0.005)

Striatum (left; caudate putamen; caudal part) and secondary somatosensory cortex (left)

【Fig. 2b】

Striatum (left; caudate putamen; rostral part)

【Fig. 2c】

Insular cortex (left)

【Fig. 2d】

Hippocampus (right; rostral)
Striatum (left; caudate putamen; caudal part)

【Fig. 3a】

Positive interaction with paw withdrawal threshold (p < 0.005)

Cingulate cortex

【Fig. 3b】

Positive interaction with paw withdrawal threshold (p < 0.005)

Primary somatosensory cortex
(right; trunk region)

Primary somatosensory cortex, right;
trunk region

$r = 0.8624$
$p = 0.0013$

【Fig. 3c】

● Positive interaction with paw withdrawal threshold (p < 0.005)

Hypothalamus (right; posterior nucleus)
Hippocampus (right; caudal)

Hypothalamus, right;
posterior nucleus

r = 0.8650
p = 0.0012

Hippocampus, right; caudal

r = 0.8541
p = 0.0017

【Fig. 3d】

Positive interaction with paw withdrawal threshold (p < 0.005)

Primary somatosensory cortex
(left; trunk region, dysgranular zone, and barrel field
right; trunk region)

Primary somatosensory cortex, left;
trunk region

r = 0.8053
p = 0.0049

【Fig. 3e】

Positive interaction with paw withdrawal threshold (p < 0.005)

Secondary somatosensory cortex
(right)

+0.6

Secondary somatosensory cortex, right

r = 0.7970
p = 0.0058

BP$_{ND}$

Paw withdrawal threshold (g)

【Fig. 3f】

● Positive interaction with paw withdrawal threshold (p < 0.005)

Primary somatosensory cortex
(right; hindlimb and forelimb region)

Primary somatosensory cortex, right
hindlimb region

r = 0.7801
p = 0.0078

【Fig. 4a】

【Fig. 4b】

Sham group

【Fig. 4c】

SNL group, normalized

【Fig. 4d】

Sham group, normalized

【Fig. 4e】

Regressed mGluR5 levels
(SNL group, normalized)

【Fig. 5a】

【Fig. 5b】

【Fig. 6a】

【Fig. 6b】

【Fig. 6c】

【Fig. 6d】

【Fig. 6e】

【Fig. 6f】

## INTERNATIONAL SEARCH REPORT

| International application No. |
|---|
| PCT/KR2019/006449 |

| A. CLASSIFICATION OF SUBJECT MATTER |
|---|

*A61B 5/00(2006.01)i, G01N 33/68(2006.01)i, G01N 33/60(2006.01)i*

According to International Patent Classification (IPC) or to both national classification and IPC

| B. FIELDS SEARCHED |
|---|

Minimum documentation searched (classification system followed by classification symbols)

A61B 5/00; A61B 10/00; A61B 5/0408; A61B 5/0476; A61B 5/05; A61K 51/00; A61K 51/04; G01N 27/327; G01R 33/48; G01N 33/68; G01N 33/60

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: pain, measure, tomography, template, compare

| C. DOCUMENTS CONSIDERED TO BE RELEVANT | | |
|---|---|---|
| Category* | Citation of document, with indication, where appropriate, of the relevant passages | Relevant to claim No. |
| Y | US 2016-0054409 A1 (WAGER, Tor et al.) 25 February 2016<br>See paragraphs [27], [35]-[43], [151] and claim 1. | 1-11 |
| A | | 12-15 |
| Y | KR 10-1579496 B1 (KOREA INSTITUTE OF SCIENCE AND TECHNOLOGY)<br>23 December 2015<br>See paragraphs [03], [05], [100]-[105] and figures 4, 5. | 1-11 |
| A | JP 5642536 B2 (NEW YORK UNIVERSITY) 17 December 2014<br>See the entire document. | 1-15 |
| A | US 6907280 B2 (BECERRA et al.) 14 June 2005<br>See the entire document. | 1-15 |
| A | JP 2009-544341 A (WARSAW ORTHOPEDIC, INC.) 17 December 2009<br>See the entire document. | 1-15 |

| ☐ Further documents are listed in the continuation of Box C. | ☒ See patent family annex. |
|---|---|

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 02 SEPTEMBER 2019 (02.09.2019) | **03 SEPTEMBER 2019 (03.09.2019)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea | |
| Facsimile No. +82-42-481-8578 | Telephone No. |

Form PCT/ISA/210 (second sheet) (January 2015)

**INTERNATIONAL SEARCH REPORT**
Information on patent family members

International application No.

**PCT/KR2019/006449**

| Patent document cited in search report | Publication date | Patent family member | Publication date |
|---|---|---|---|
| US 2016-0054409 A1 | 25/02/2016 | WO 2014-169060 A1 | 16/10/2014 |
| KR 10-1579496 B1 | 23/12/2015 | None | |
| JP 5642536 B2 | 17/12/2014 | CA 2682955 A1 | 16/10/2008 |
| | | CN 101677775 A | 24/03/2010 |
| | | CN 101677775 B | 08/08/2012 |
| | | EP 2131733 A2 | 16/12/2009 |
| | | JP 2010-523226 A | 15/07/2010 |
| | | US 2008-0249430 A1 | 09/10/2008 |
| | | US 2016-0302720 A1 | 20/10/2016 |
| | | US 9402558 B2 | 02/08/2016 |
| | | WO 2008-124566 A2 | 16/10/2008 |
| | | WO 2008-124566 A3 | 04/12/2008 |
| US 6907280 B2 | 14/06/2005 | AU 1943301 A | 12/06/2001 |
| | | AU 5304001 A | 15/10/2001 |
| | | CA 2403974 A1 | 11/10/2001 |
| | | EP 1237476 A1 | 11/09/2002 |
| | | EP 1272105 A2 | 08/01/2003 |
| | | JP 2003-528679 A | 30/09/2003 |
| | | US 2002-0042563 A1 | 11/04/2002 |
| | | US 2002-0058867 A1 | 16/05/2002 |
| | | US 2008-0039737 A1 | 14/02/2008 |
| | | WO 01-39664 A1 | 07/06/2001 |
| | | WO 01-39664 A8 | 04/10/2001 |
| | | WO 01-74240 A2 | 11/10/2001 |
| | | WO 01-74240 A3 | 23/05/2002 |
| JP 2009-544341 A | 17/12/2009 | EP 2032024 A2 | 11/03/2009 |
| | | US 2007-0287991 A1 | 13/12/2007 |
| | | WO 2007-146616 A2 | 21/12/2007 |
| | | WO 2007-146616 A3 | 10/04/2008 |
| | | WO 2007-146616 B1 | 12/06/2008 |

Form PCT/ISA/210 (patent family annex) (January 2015)